# EUROPEAN PATENT APPLICATION

(11) **EP 1 088 557 A1**
(43) Date of publication of application: **04.04.2001**
(21) Application number: 99500176.5
(22) Date of filing: 28.09.1999
(51) Int. Cl.: A61K 39/00

(54) **Immunisation against cancer with mutated p53**

(71) Applicant: Plumley, Kevin More, 36203 Vigo (ES)
(72) Inventor: Plumley, Kevin More, 36203 Vigo (ES)

(57) **Abstract**

Immunisation against cancer is proposed by damaging p53 material relevant to cancer, with all the means by which mutation occurs in p53 in natural circumstances, then introducing it into others in order to cause their immune system to produce antibodies against the damaged alien p53, so that the individuals so treated will have protection against their own p53 material, defective now or in the future, which their own immune system otherwise would not recognise. This will decrease or eliminate the likelihood of cancers occurring in treated individuals.

## Description

Immunisation against cancer is proposed by damaging the genetic material of others which acts crucially as the brake in cell-subdivision (p53), then using it to cause other individuals' immune systems to recognise similar deficiencies as they arise in their own material, suppressing their effects, and thus decreasing or eliminating the likelihood of cancers occurring in those so treated.

Cancers at present are removed by excision or by chemical treatment, but as yet no effective attempt seems to have been made to counter the basic condition which initially permits the cancer to commence. Thus the object of this patent application is a technique in which the following procedure is undertaken to do just that.

P53 is the designation given to the genetic component which acts as a brake in cell-subdivision. If it is absent or damaged, cells can replicate without control, and in certain circumstances can become cancer. All cancer - and pre-cancer - cells are deficient in p53.

Unfortunately, the individual's immune system does not recognise this deficiency in its own cells as a fault - It is you, so to speak So the individual cannot eradicate such cells. And if or when their uncontrolled replication becomes cancer, your immune system still insists that - it is just you.

The immune system of the individual, as we have seen, will not recognise its host's own mutated cellular deficiencies. Therefore we take a few of my cells, say, and intentionally artificially mutate - by damage - their p53 in a variety of ways, and then replicate this material - differentiated or undifferentiated - or even simply its damaged p53 - in abundance by polymerase chain reaction. Then a few cells of this material are injected into other individuals, irrespective of whether they yet have cells with damaged or deficient p53. Logically, the following will then have to happen: their immune system will wake to the fact that alien cells - mine - are present, and will manufacture antibodies against them - including, specifically, against the p53-deficiency factor. These antibodies then will go to combat any cells deficient in p53 that their bodies contain, mine or theirs, or will wait for such cells to appear by natural mischance in the future, and act against them then.

In this way, cells - present now or occurring in the future - which otherwise potentially might result in future cancer, will be suppressed, and the potential cancers will not occur. This can equally be applied to other species, too.

It is not known how effective this technique will be against established cancers. The 'adept' (antibody-directed enzyme-pro-drug therapy) treatment is already in use in destroying existing cancers, but the suggested use either of others' cells with intentionally impaired p53 or even simply of the damaged p53 itself - is offered as a simple method of providing immunity and thus preventing cancers from occurring at all.

To alter the p53 in the intended vaccine it is necessary to vary the constituents ofthe p53 in as many ways as possible, to provide the widest antibody array against as many variations of naturally-occurring p53 damage as possible. It is emphasised that 'damage' refers to causing alteration in the p53 material beyond the natural variations that may be found between the p53 material of different individuals. This is done by subjecting the p53 to exposure to gamma rays, x-rays, ultra-violet light, alpha and beta and other high energy particles, heat treatment, and contact with relevant moulds such as the nut-moulds which promote liver cancer, and viruses such as those causing cervical cancer - i.e. in all the ways by which the p53 naturally mutates to become abnormal and so produce cancers. The resulting damaged p53 may then be multiplied as differentiated or undifferentiated material as required, and either used immediately or dried in trehalose and stored at room temperature for later use.

It is expected and supposed that the cancellation of this underlying factor crucial to all cancers may well disrupt the entire cancer syndrome and therefore alone be sufficient to prevent all cancers.

It will be necessary to remedy an increasing innate tendency to produce p53-deficient cells in descendants of those who, rather than dying as a result of the cancers that p53-deficient cells would otherwise produce - thus taking the problem into the grave with them - therefore will instead live and produce progeny, but it is better to cross that bridge when we come to it.

This technique and its implementation will be cheap and should be available to everyone, a mere few cell cultures being needed with damaged or deficient p53 to be endlessly and cheaply replicated, a single culture being effective to all except its donor or identical twin.

## Claims

1. Immunisation against cancer involves the artificial mutation, by exposure to gamma rays, of a factor crucial to the cancer syndrome - p53, then its introduction into others to cause their immune system to recognise and suppress the same condition in its host, thus decreasing or eliminating the likelihood of producing cancer in those so treated.

2. Immunisation against cancer involves the artificial mutation, by exposure to gamma rays as in Claim 1, and / or to x-rays, of a factor crucial to the cancer syndrome - p53, then its introduction into others to cause their immune system to recognise and suppress the same condition in its host, thus decreasing or eliminating the likelihood of producing cancer in those so treated.

3. Immunisation against cancer involves the artificial mutation, by exposure to gamma- and / or x-rays as in Claims 1 and 2, and / or to ultra-violet light, of a factor crucial to the cancer syndrome - p53, then its introduction into others to cause their immune system to recognise and suppress the same condition in its host, thus decreasing or eliminating the likelihood of producing cancer in those so treated.

4. Immunisation against cancer involves the artificial mutation, by exposure to gamma- and / or x -rays and / or to ultra-violet light, as in Claims 1 to 3, and / or to irradiation with alpha particles, of a factor crucial to the cancer syndrome - p53, then its introduction into others to cause their immune system to recognise and suppress the same condition in its host, thus decreasing or eliminating the likelihood of producing cancer in those so treated.

5. Immunisation against cancer involves the artificial mutation, by exposure to gamma- and / or x -rays and / or to ultra-violet light and / or to irradiation with alpha particles, as in Claims 1 to 4, and / or with beta particles, of a factor crucial to the cancer syndrome - p53, then its introduction into others to cause their immune system to recognise and suppress the same condition in its host, thus decreasing or eliminating the likelihood of producing cancer in those so treated .

6. Immunisation against cancer involves the artificial mutation, by exposure to gamma- and / or x -rays and / or to ultra-violet light and / or to irradiation with alpha and / or beta particles, as in Claims 1 to 5, and / or with other high-energy particles, of a factor crucial to the cancer syndrome - p53, then its introduction into others to cause their immune system to recognise and suppress the same condition in its host, thus decreasing or eliminating the likelihood of producing cancer in those so treated .

7. Immunisation against cancer involves the artificial mutation, by exposure to gamma- and / or x -rays and / or to ultra-violet light and / or to irradiation with alpha and / or beta and / or other high-energy particles, as in Claims 1 to 6, and / or to treatment with cancer-causing moulds, of a factor crucial to the cancer syndrome - p53, then its introduction into others to cause their immune system to recognise and suppress the same condition in its host, thus decreasing or eliminating the likelihood of producing cancer in those so treated.

8. Immunisation against cancer involves the artificial mutation, by exposure to gamma- and / or x -rays and / or to ultra-violet light and / or to irradiation with alpha and / or beta and / or other high-energy particles and / or to treatment with cancer-causing moulds, as in Claims 1 to 7, and / or with cancer-causing viruses, of a factor crucial to the cancer syndrome - p53, then its introduction into others to cause their immune system to recognise and suppress the same condition in its host, thus decreasing or eliminating the likelihood of producing cancer in those so treated.

9. Immunisation against cancer involves the artificial mutation, by exposure to gamma- and / or x -rays and / or to ultra-violet light and / or to irradiation with alpha and / or beta and / or other high-energy particles and / or to treatment with cancer-causing moulds and / or cancer-causing viruses, as in Claims 1 to 8, and / or with heat treatment, of a factor crucial to the cancer syndrome - p53, i.e. by all influences found in the natural mutation ofp53, then its introduction into others to cause their immune system to recognise and suppress the same condition in its host, thus decreasing or eliminating the likelihood of producing cancer in those so treated.
